# EUROPÄISCHE PATENTANMELDUNG

(11) **EP 1 980 277 A1**
(43) Veröffentlichungstag der Anmeldung: **15.10.2008**
(21) Anmeldenummer: 07005528.0
(22) Anmeldetag: 17.03.2007
(51) Int. Cl.: A61L 29/08, A61L 31/10

(54) **Gastroenterologisches Medizinprodukt, insbesondere Stent für den Gallen- oder Pankreasgang**

(71) Anmelder: Hildebrandt, Peter, 90455 Nürnberg (DE)
(72) Erfinder: Hildebrandt, Peter, 90455 Nürnberg (DE)
(74) Vertreter: Hübner, Gerd

(57) **Zusammenfassung**

Ein gastroenterologisches Medizinprodukt, insbesondere ein Stent für den Gallen- oder Pankreasgang, weist einen im wesentlichen röhrenförmigen, eigenstabilen Träger (1) auf, der mit einer an der Trägeroberfläche (2, 3) gebundenen Spacer-Lage und einer an der Spacer-Lage gebundenen Lage aus einem elektronegativen Glykosaminoglykan versehen ist.

## Beschreibung

Die Erfindung betrifft ein gastroenterologisches Medizinprodukt mit einem im Wesentlichen röhrenförmigen, eigenstabilen Träger.

Solche Medizinprodukte werden im Fachjargon üblicherweise als Stents, Prothesen oder Katheter bezeichnet. Gastroenterologische Stents und Prothesen werden in der Regel zur Aufweitung und Offenhaltung von krankhaft verschlossenen oder im Durchmesser verengten Gängen und Wegen im Körper verwendet. Im Bereich der Katheter sind als Anwendungsbeispiele für die angegebenen gastroenterologischen Medizinprodukte nasale und perkutane Ernährungssonden zu nennen.

Im Folgenden soll der Einfachheit halber bei der Erörterung des Standes der Technik und der Erfindung auf die bereits erwähnten Stents beispielhaft eingegangen werden:

Gastroenterologische Stents sind für Patienten mit Gallen- und Pankreasgangstörungen unverzichtbare Implantate. Eine große Auswahl an Stent-Ausgestaltungen und Materialien sind heute bereits verfügbar. Ausgestaltung und Material richten sich in der Regel nach dem Einsatzort des Stents und der verwendeten Implantationstechnik. So sind beispielsweise röhrenförmige Träger, die aus einem expandierbaren Gittermaterial gefertigt sind, üblich. Häufig werden auch schlauchartige Stents mit geschlossener Wand aus Polymermaterial verwendet. Der Stent wird endoskopisch in den Gallen- oder Pankreasgang eingeführt. Er verbleibt am Implantationsort und dient zur Abstützung gegen eine abermalige Verengung.

Als schwerwiegendes Problem treten bei allen bisher eingesetzten und getesteten Materialien für gastroenterologische Medizinprodukte bereits bei mehrtägigem Kontakt mit Galle, Pankreassekret, Magen- oder Darminhalt Bakterienbelag und Ablagerungen - sogenannter Biofilm - an der Oberfläche des Medizinproduktes auf. Dies führt zu schwerwiegenden Funktionsstörungen, wie Infektionen und einem teilweisen oder vollständigen Verschluß des röhrenförmigen Medizinproduktes, was den medizinischen Erfolg nachhaltig gefährdet. Zahlreiche Untersuchungen haben sich in Hinsicht auf dieses Problem mit verschiedenen medizintechnischen Werkstoffen befasst. Es wurde jedoch bei noch keinem Material eine wirklich dauerhafte Resistenz gegen die genannten Effekte nachgewiesen.

Ein Ansatz aus dem Stand der Technik zur Lösung der Biofilmproblematik ist der US 6 228 393 B1 zu entnehmen. In dieser Druckschrift ist eine Beschichtung offenbart, die Wirkstoffe wie beispielsweise Antibiotika freigibt. Dadurch soll die Bildung eines Bakterienfilms an der Oberfläche verhindert werden. Der Stand der Technik offenbart zahlreiche weitere Patente, die auf dem Prinzip der Wirkstofffreigabe basieren. Dabei wird jedoch ein Reservoir an Wirkstoffen benötigt, das zwangsläufig beschränkt ist. Ist dieses Reservoir aufgebraucht, so nimmt die Oberfläche wieder ihre ursprünglichen Eigenschaften bezüglich der Anfälligkeit für bakterielle Besiedlung an. Dies lässt die Anwendung bei längeren Liegezeiten als wenig sinnvoll erscheinen.

Ein weiteres Beispiel für entsprechende Maßnahmen aus dem Stand der Technik ist dem US-Patent 6 361 567 B1 entnehmbar. In dieser Druckschrift ist eine antimikrobielle Beschichtung zur Anwendung bei medizinischen Implantaten offenbart. Das Wirkungsprinzip der Beschichtung basiert auf der Verwendung von antimikrobiellen Metallionen, wie Silber, Gold oder einer Kombination aus beiden. Der Stand der Technik offenbart zahlreiche weitere Patente, die auf dem Prinzip der antimikrobiellen Eigenschaften von Metallionen basieren. Wie sich jedoch gezeigt hat, ist die mit diesem Stand der Technik erreichbare Eindämmung von Bakterienbesiedlung nach wie vor verbesserungsbedürftig.

EP 0 890 367 B1 offenbart eine Glykosaminoglykan Beschichtung auf urologischen Implantaten im Kontakt mit Urin. In dieser Umgebung wirken die Glykosaminoglykane als Inhibitoren, d.h. durch Adsorption an Moleküle und Kristalloberflächen im Urin und blockieren so deren Bindungsstellen. Damit wird das Anwachsen größerer Kristallstrukturen verhindert. Dabei ist die Wirkung eines Inhibitors meist auf eine oder wenige Teilchensorten beschränkt. So bindet z. B. Nephrocalcin an Kalzium-Ionen und Heparin an Oxalat-Ionen sowie -Kristalliten als Inhibenten. Eine kovalente Bindung von Inhibitoren an das Substrat erzeugt daher eine Schicht, an der Urinkomponenten so gebunden werden, dass sich keine weiteren Komponenten anlagern können und Kristallwachstum blockiert wird. Dieser Mechanismus ist für den Einsatz eines gastroenterologischen Implantats insbesondere für den Gallen- und Pankreasgang in keiner Weise relevant.

Der Erfindung liegt nun die Aufgabe zugrunde, ein für gastroenterologische Zwecke optimiertes Implantat bereitzustellen, bei dem eine bakterielle Besiedlung und Biofilmbildung wirkungsvoll unterbunden sind.

Die Erfindung schlägt dementsprechend ein gastroenterologisches Medizinprodukt mit einem im Wesentlichen röhrenförmigen, eigenstabilen Träger vor, bei dem laut Patentanspruch 1 an der Oberfläche des Trägers eine Spacer-Moleküllage gebunden ist, an der wiederum eine Lage aus einem elektronegativen Glykosaminoglykan gebunden ist.

Der vorliegenden Erfindung liegen im Gegensatz zu den geschilderten kristallisationsinhibierenden Mechanismen bei Urinalstents biomedizinische Vorgänge zur Unterbindung der bakteriellen Besiedlung und Biofilmbildung im Kontakt mit Galle, Pankreassekret, Magen- oder Darminhalt als Ansatz zugrunde:

Die Bildung von Biofilm läuft im Normalfall in folgenden Schritten ab: Der erste Schritt ist die Anlagerung von Mikroorganismen an die Implantatoberfläche, gefolgt von deren stabilen Verankerung durch mikrobische Adhäsion und Exopolymer Produktion. Danach ist meist ein starkes Wachstum der Mikroorganismen zu beobachten.

Der Biofilm ist in den meisten Fällen aus drei Schichten aufgebaut: Der Verbindungsfilm sichert die Verankerung an die Implantatoberfläche, die mittlere Biofilmschicht besteht aus Mikroorganismen und der Oberflächenfilm stellt die äußere Oberfläche des Biofilms dar, an der planktonische Organismen mit der Umgebung ausgetauscht werden können.

Glykosaminoglykane sind zum einen Stoffe, die natürlich in Körperflüssigkeiten gelöst vorkommen und zum anderen ein Bestandteil der obersten Zellschichten von Gefäßen, Wegen und Gängen im Körper. Sie verfügen über eine starke Elektronegativität und stoßen damit zelluläre Organismen ab. Damit sind Glykosaminoglykane überraschender Weise auch exzellent für die Beschichtung von Implantaten geeignet, die vor Biofilmbildung im Kontakt mit Galle, Pankreassekret, Magen- oder Darminhalt geschützt werden sollen.

Wie im Hinblick auf die Erfindung festgestellt wurde, reicht dabei eine direkte Bindung von Glykosaminoglykanen an der Trägeroberfläche nicht aus. Die direkte Bindung führt nämlich zu einer Abschwächung des elektronegativen Feldes, was die Wirksamkeit stark abschwächt. Das Einfügen des beanspruchten Spacers zwischen der Trägeroberfläche und der Glykosaminoglykan-Lage vergrößert deren Abstand und kann damit die oben erläuterte Abschwächung des elektronegativen Feldes vermeiden. Weiterhin ermöglicht diese Methode der Anbindung die Langzeitstabilität der Beschichtung und damit auch deren Wirkung.

Gemäß bevorzugten Ausführungsformen kann der Träger einerseits aus einem Polymer, wie Silikon, Polyurethan, Polyethylen oder dergleichen oder andererseits aus Metallen oder Metalllegierungen, wie medizinischem Edelstahl, Titan, Nickel-Titan oder dergleichen jeweils mit einer amorphen Siliziumkarbid-Beschichtung als aktiver Substratoberfläche bestehen. Auch eine Ausbildung aus einem Metallgerüst mit einer Polymer-Beschichtung ist möglich. Je nach Materialbeschaffenheit des Trägers und seiner Substratoberfläche wird auch die Auswahl für die chemischen Komponenten der Spacer-Lage getroffen.

Bevorzugterweise wird als Glykosaminoglykan-Lage eine auf der Spacer-Lage immobilisierte Heparin-Lage verwendet. Die Heparin-Lage wird dabei mittels einer kovalenten Bindung auf der Substratoberfläche angelagert und immobilisiert.

Zusammenfassend haben experimentelle Untersuchungen mit erfindungsgemäß ausgerüsteten gastroenterologischen Stents, bei denen ein Polyethylen-Träger eingesetzt wurde, eine starke Unterdrückung der bakteriellen Besiedlung und Biofilmbildung im Kontakt mit Gallen- und Pankreassekret ergeben. Durch die experimentellen Untersuchungen konnte auch eine ausreichende Langzeitstabilität der Heparin-Bindung an der Spacer-Lage nachgewiesen werden.

Weitere Merkmale, Einzelheiten und Vorteile des Erfindungsgegenstandes sind der nachfolgenden Beschreibung entnehmbar, in der ein Ausführungsbeispiel des Erfindungsgegenstandes anhand der beigefügten Zeichnung näher erläutert wird. Es zeigt die

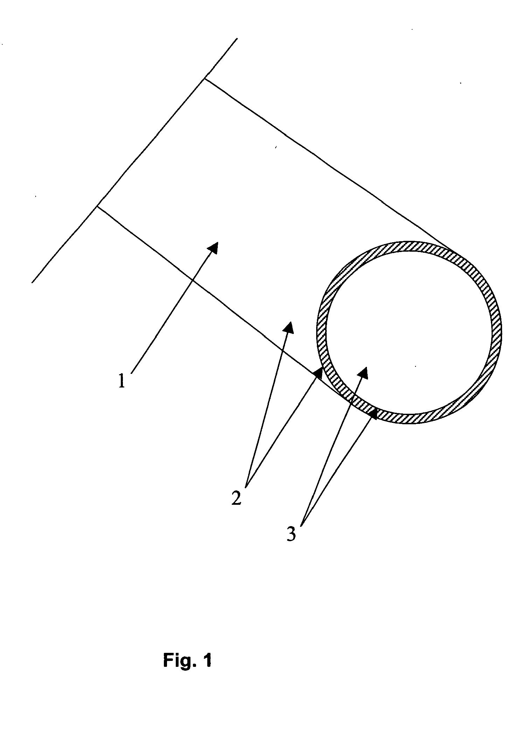

Fig. 1 eine schematische, geschnittene Perspektivdarstellung eines gastroenterologischen Stents.

Der gezeigte Stent weist einen Träger 1 auf, der beispielsweise aus Polyethylen besteht. An dessen äußerer Oberfläche 2 und innerer Oberfläche 3 ist eine nicht näher dargestellte Schicht eines Glykosaminoglykans kovalent über einen Spacer gebunden.

Nachfolgend wird in Form einer Auflistung einzelner Verfahrensschritte ein beispielhaftes Beschichtungsverfahren zur Immobilisierung von Heparin auf einer Probe in Form einer Polyethylenoberfläche des Trägers des Stents wiedergegeben:
- Probe für 2 Minuten in konzentrierte Schwefelsäure und Kaliumpermanganat einlegen.
- Probe mehrfach in deionisiertem Wasser spülen.
- Probe zur Anbindung der Spacer-Lage für 5 Minuten in eine wässrige Polyethylenimin-Lösung einlegen.
- Probe mehrfach in deionisiertem Wasser spülen.
- Probe zur Bildung der Glykosaminoglykanlage auf der Spacer-Lage für 2 Stunden in eine wässrige Heparinlösung mit Cyanoborhydrid einlegen.
- Probe mehrfach in deionisiertem Wasser spülen.

Für die Spacer-Lage sind verschiedene Alternativen möglich. Sokann die Spacer-Lage auf der Basis einer Propylsiloxyl-Verbindung, wie z.B. einer partiell substituierten 3-(Adipinsäureamido)Propylsiloxyl-Verbindung, gebildet sein. Auch die Verwendung einer photoaktiven Benzophenon-Verbindung, wie z.B. einer Fmoc-p-Bz-Phe-OH-Lösung in N,N'-Dimetthylformamid als photoaktiver Benzophenonverbindung, ist vorzusehen.

Die Glykosaminoglykanlage kann auch aus einer auf der Spacer-Lage immobilisierten Lage aus einem synthetischen Heparinderivat - gegebenenfalls in Kombination verschiedener Glykosaminoglykane - gebildet sein.

## Patentansprüche

1. Gastroenterologisches Medizinprodukt, insbesondere ein Stent für den Gallen- oder Pankreasgang, mit einem im wesentlichen röhrenförmigen, eigenstabilen Träger (1),
**gekennzeichnet durch**
eine an der Trägeroberfläche (2, 3) gebundene Spacer-Lage und eine an der Spacer-Lage gebundene Lage aus einem elektronegativen Glykosaminoglykan.

2. Gastroenterologisches Medizinprodukt nach Anspruch 1, **dadurch gekennzeichnet, dass** der Träger (1) aus einem Polymer, insbesondere Silikon, Polyurethan, Polyethylen, Polyvinylchlorid oder dergleichen besteht.

3. Gastroenterologisches Medizinprodukt nach Anspruch 1, **dadurch gekennzeichnet, dass** der Träger (1) aus einem eigenstabilen Gerüst, insbesondere aus Metall, mit einer Beschichtung aus einem Polymer, insbesondere Silikon, Polyurethan, Polyvinylchlorid oder dergleichen besteht.

4. Gastroenterologisches Medizinprodukt nach Anspruch 1, **dadurch gekennzeichnet, dass** der Träger (1) aus Tantal, einer Titanlegierung, medizinischem Edelstahl oder pyrolytischem Kohlenstoff jeweils mit einer amorphen Siliziumkarbid-Beschichtung als aktivierter SubstratOberfläche besteht.

5. Gastroenterologisches Medizinprodukt nach Anspruch 2 oder 3, **dadurch gekennzeichnet, dass** die Spacer-Lage auf der Basis einer Polyethylenimin-Verbindung gebildet ist.

6. Gastroenterologisches Medizinprodukt nach Anspruch 2 oder 3, **dadurch gekennzeichnet, dass** die Spacer-Lage auf der Basis einer Propylsiloxyl-Verbindung gebildet ist.

7. Gastroenterologisches Medizinprodukt nach Anspruch 6, **dadurch gekennzeichnet, dass** die Spacer-Lage aus einer partiell substituierten 3-(Adipinsäureamido)Propylsiloxyl-Verbindung gebildet ist.

8. Gastroenterologisches Medizinprodukt nach Anspruch 4, **dadurch gekennzeichnet, dass** die Spacer-Lage auf der Basis einer photoaktiven Benzophenon-Verbindung gebildet ist.

9. Gastroenterologisches Medizinprodukt nach Anspruch 8, **gekennzeichnet durch** eine Fmoc-p-Bz-Phe-OH-Lösung in N,N'-Dimetthylformamid als photoaktive Benzophenonverbindung.

10. Gastroenterologisches Medizinprodukt nach einem der Ansprüche 1 bis 9, **dadurch gekennzeichnet, dass** die Glykosaminoglykan-Lage aus einer auf der Spacer-Lage immobilisierten Heparin-Lage gebildet ist.

11. Gastroenterologisches Medizinprodukt nach einem der Ansprüche 1 bis 9, **dadurch gekennzeichnet, dass** die Glykosaminoglykan-Lage aus einer auf der Spacer-Lage immobilisierten Lage aus einem synthetischen Heparinderivat gebildet ist.

12. Gastroenterologisches Medizinprodukt nach einem der Ansprüche 1 bis 9, **dadurch gekennzeichnet, dass** die Glykosaminoglykan-Lage aus einer auf der Spacer-Lage immobilisierten Lage aus einer Kombination verschiedener Glykosaminoglykane gebildet ist.
